# EUROPÄISCHE PATENTSCHRIFT

(11) **EP 1 074 229 B1**
(45) Veröffentlichungstag und Bekanntmachung des Hinweises auf die Patenterteilung: **05.10.2005**
(21) Anmeldenummer: 00115029.1
(22) Anmeldetag: 25.07.2000
(51) Int. Cl.: A61F 2/38, A61F 2/30

(54) **Verfahren zur Herstellung einer Endoprothese als Gelenkersatz bei Kniegelenken**
Method of manufacturing a knee joint replacing endoprosthesis
Méthode de fabrication d'une endoprothèse pour le remplacement de l'articulation du genou

(30) Priorität: 04.08.1999 DE 19936682
(43) Veröffentlichungstag der Anmeldung: 07.02.2001
(73) Patentinhaber: Schuster, Luis, Dr.med., 80639 München (DE)
(72) Erfinder: Schuster, Luis, Dr.med., 80639 München (DE); Schuster, Christoph, Dr.med., 82031 Grünwald (DE)
(74) Vertreter: Müller, Frank Peter

(56) Entgegenhaltungen:
- EP-A- 0 255 797
- DE-A- 4 434 539
- DE-A- 10 003 533

## Beschreibung

Die Erfindung bezieht sich auf ein Verfahren zur Herstellung einer Endoprothese als Gelenkersatz bei Kniegelenken sowie auf ein Operationsset, das zur Durchführung von Kniegelenk-Operationen unter Verwendung einer Endoprothese geeignet ist, die gemäß einem solchen Verfahren hergestellt wurde.

Eine Kniegelenk-Operation wird von dem behandelnden Arzt in aller Regel dann in Betracht gezogen, wenn der Patient über nicht mehr duldsame Knieschmerzen und Behinderungen als Folge bspw. einer rheumatischen Arthritis oder auch sonstiger Gelenkerkrankungen klagt. Die Operation wird dabei meistens mit einer Vielzahl von Stufen durchgeführt, um eine Anpassung an einzelne Gelenkformteile zu erhalten, die industriell gefertigt wurden und in verschiedenen Größen und Formgebungen zur Verfügung stehen. Die für die Operation letztlich ausgewählten Teile erfahren eine Befestigung an gesägten Flächen hauptsächlich des femoralen Condylus, des distalen Femur, der proximalen Tibia und der Patella. Dabei wird für die Befestigung eine senkrechte Ausrichtung zu einer Achse angestrebt, die vor der Operation bspw. mittels einer Röntgenfilmaufnahme und eines Marknagel-Ausrichtungssystems für die gerade Verbindungslinie zwischen der Hüftmitte, dem Knie und dem Fußknöchel erhalten wird.

Die Implantation solcher mehrteiliger Kniegelenk-Endoprothesen ist relativ aufwendig. Unter Berücksichtigung auch des häufig recht unterschiedlichen Wuchses der Patienten ist dabei auch nachteilig, daß bei einer solchen nur angenäherten Wiederherstellung der Verhältnisse eines gesunden Kniegelenks auch Komplikationen auftreten, die auf die Mechanik der implantierten Komponenten der Endoprothese zurückzuführen sind und bspw. ein vorderes Kniegelenk-Schmerzsyndrom ergeben, welches durch ein Fehlgleiten der Kniescheibe mit einer unphysiologischen Belastung des Oberschenkel-Kniescheibengleitgelenks und des Oberchenkel-Schienbeingelenks verursacht wird.

Zur Vermeidung derartiger Komplikationen, die häufig eine wiederholte Implantation einer neuen Endoprothese mit dem begleitenden Nachteil einer Resektion von weiteren Knochenteilen erfordern, ist gemäß der DE 44 34 539 C2 bereits ein Verfahren bekannt, bei welchem vor der eigentlichen Operation mit Mitteln der Computer-Tomographie oder der Kernspintomographie ein tomographisches Bild von dem geschädigten Kniegelenk angefertigt wird. Dieses präoperative Bild erfährt anschließend eine Korrektur hinsichtlich einer Annäherung der Konturen wenigstens am Femur und an der Tibia bei dem geschädigten Kniegelenk an die Konturen bei einem gesunden Kniegelenk. Nach einer solchen Korrektur wird ein virtuelles postoperatives Bild von dem geschädigten Kniegelenk angefertigt, welches für einen Vergleich mit dem präoperativen Bild zur Verfügung steht. Aus diesem Vergleich wird dann noch ein Subtraktionsbild angefertigt, welches aus der Gegenüberstellung des korrigierten präoperativen Bildes und des postoperativen Bildes den Unterschied der Konturen wenigstens am Femur und an der Tibia bei den beiden Bilder ermitteln lässt. Auf der Basis dieses Subtraktionsbildes können dann die femoralen und tibialen Komponenten einer Endoprothese hergestellt werden, die somit individuell an die Verhältnisse des geschädigten Kniegelenks sehr eng angepasst sind.

Die bei diesem Verfahren geübte Herstellung einer Endoprothese auf der Basis eines besonderen Subtraktionsbildes erfordert auch wegen der Einbeziehung des dafür noch benötigten postoperativen Bildes zusätzlich zu dem korrigierten präoperativen Bild des betreffenden Gelenkteils einen höheren Computeraufwand. Es können sich deshalb vermehrt Fehlerquellen ergeben bei der späteren Umsetzung der letzlich für die Subtraktionsbilder gespeicherten Daten für die dreidimensionale Herstellung der Endoprothese-Komponenten.

Der Erfindung liegt daher die Aufgabe zugrunde, das mit solchen tomographischen Bildern für die Herstellung einer Endoprothese arbeitende Verfahren derart zu gestalten, daß dem behandelnden Arzt ein Operationsset zur Durchführung von Kniegelenk-Operationen zur Verfügung gestellt werden kann, bei welchem die dafür wenigstens benötigten femoralen und tibialen Komponenten der Endoprothese auch ohne einen Rückgriff auf ein Vergleichsbild zur Ermittlung der Unterschiede hergestellt wurden, die für das geschädigte Kniegelenk zum Zeitpunkt vor und nach der Operation vorgegeben wurden.

Diese Aufgabe wird bei einem Verfahren zur Herstellung einer Endoprothese als Gelenkersatz bei Kniegelenken erfindungsgemäß gelöst mit dem Merkmalen des Patentanspruches 1. Weitere Verfahrensmerkmale, mit welchen die Erfindung zweckmäßig ausgebildet wird, ergeben sich aus den Unteransprüchen, die für eine vorteilhafte Anwendung des Verfahrens auch auf ein Operationsset Bezug nehmen, welches sich für die Durchführung von Kniegelenk-Operationen als besonders zweckmäßig erweist.

Gemäß der vorliegenden Erfindung wird somit zuerst ein tomographisches Bild von dem geschädigten Kniegelenk angefertigt. Dieses Bild kann mittels der Computer-Tomographie oder auch bevorzugt mittels der Kernspintomographie erhalten werden, welche die Gelenkkonturen besonders scharf abgrenzen lässt.

Das tomographische Bild des geschädigten Kniegelenks wird dann anschließend korrigiert für eine Annäherung der Konturen wenigstens der femoralen und der tibialen Gelenkteile des geschädigten Kniegelenks an deren Konturen bei einem gesunden Kniegelenk. Bei dieser somit virtuellen Korrektur in einem bevorzugt gestreckten Zustand des Knies werden die hintere und die untere Gelenkfläche des Femurs und die gesamte Schienbeinfläche der Tibia für ein erwünschtes Erreichen von mehr oder weniger idealen Konturen bei dem späteren Einsetzen der Endoprothese während der eigentlichen Kniegelenk-Operation nachgefahren. Die Korrektur kann dabei an dem ursprünglich angefertigten Bild manuell vorgenommen werden oder es ist alternativ auch möglich, die Korrektur mit dem Bild einer Spiegelbildaufnahme eines zu dem geschädigten Kniegelenk gegenseitigen, gesunden Kniegelenks auszuführen. Auch kann diese Korrektur durch einen Vergleich mit Bildern von gesunden Kniegelenken vorgenommen werden, die mit dem geschädigten Kniegelenk vergleichbare Gelenkflächen wenigstens des Femur und der Tibia aufweisen.

Sobald diese Korrektur abgeschlossen ist, werden dann die korrigierten femoralen und tibialen Gelenkteile virtuell abgetrennt bzw. absegmentiert. Die Abtrennung wird dabei an markierten Schnittflächen vorgenommen, die bei der späteren Operation für die an den Gelenkknochen durchgeführte Abtrennung der geschädigten Gelenkteile eingehalten werden. Diese Abtrennung wird am Femur des geschädigten Kniegelenks bevorzugt mit drei unterschiedlichen Schnittebenen und an der Tibia mit bis zu zwei Schnittebenen vorgegeben. Es werden daher unmittelbar an dem geschädigten Kniegelenk orientierte bildmäßige Vorlagen erhalten, die eine mit den korrigierten femoralen und tibialen Gelenkteilen übereinstimmende Herstellung der femoralen und tibialen Komponenten einer bei der späteren Kniegelenk-Operation eingesetzten Endoprothese erlauben. Die markierten Schnittflächen können dabei bildmäßig noch ergänzt werden mit passgenauen Verankerungshilfen für diese späteren Komponenten der Endoprothese an den späteren Resektionsflächen der Gelenkknochen. Solche Verankerungshilfen können bspw. die Ausbildung von zapfenförmigen Vorsprüngen erhalten, die dann in zugeordnete Bohrkanäle der Resektionsflächen passgenau eingesetzt werden können.

Die für die Abtrennung der korrigierten femoralen und tibialen Gelenkteile markierten Schnittflächen werden außerdem für die Anfertigung eines virtuellen Bildes einer Schablone getrennt für Femur und Tibia des geschädigten Kniegelenks übernommen, die als bildmäßige Vorlage für eine mit dem Kniegelenk passgenau übereinstimmende dreidimensionale Implantationshilfe benutzt wird. In Übereinstimmung mit der Anfertigung der femoralen und tibialen Komponenten der Endoprothese wird somit auch das virtuelle Bild einer solchen Schablone direkt benutzt für die spätere dreidimensionale Herstellung einer zugehörigen Implantationshilfe für die einzelnen Komponenten der Endoprothese. Die für die Schablone übernommenen Schnittflächen werden an der Implantationshilfe als Führungsschlitze für ein oszillierendes Sägeblatt ausgebildet, mit welchem bei der späteren Operation eine Abtrennung bzw. Absegmentierung der geschädigten Gelenkteile an den Gelenkknochen vorgenommen wird. Bei der Anfertigung des virtuellen Bildes der Schablone muß deshalb auch entsprechend sorgfältig beachtet werden, daß die spätere Implantationshilfe an dem geschädigten Kniegelenk einen derart präzisen Sitz erhalten kann, daß mit dem für die Resektion der geschädigten Gelenkteile in den Führungsschlitzen geführten Sägeblatt Resektionsflächen an den Gelenkknochen erhalten werden, die genau übereinstimmen mit den Passflächen der femoralen und tibialen Komponenten der Endoprothese, welche mit den markierten Schnittflächen ihrer bildmäßigen Vorlagen vorgegeben wurden. Solche Schablonen respektive ihre entsprechenden Implantationshilfen sind daher etwa kappenförmig auszubilden, um eine Umhüllung der Schnittflächen zu erhalten, die sich daher an den Schablonen als das Negativbild der späteren Resektionsflächen der Gelenkknochen darstellen, die mittels der Implantationshilfen bei der Kniegelenk-Operation erhalten werden.

Die bildmäßigen Vorlagen für die femoralen und tibialen Komponenten einer Endoprothese und für die femoralen und tibialen Komponenten einer zugehörigen Implantationshilfe werden dann für die Herstellung von übereinstimmenden dreidimensionalen Teilen umgesetzt. Für die Umsetzung steht das sog. "Rapid-Prototyping" zur Verfügung, wobei damit sog. STL-Modelle erhalten werden, die als Vorlagen für die Herstellung der Formteile im Gußverfahren verwendet werden. Für die tibiale Komponente der Endoprothese kann dabei ein zur Anordnung an dem Gelenkknochen vorgesehenes Metallteil und ein Kunststoffteil vorgegeben werden, welches für eine mittige Anordnung zwischen dem Metallteil der tibialen Komponente und der ebenfalls als ein Metallteil ausgeführten femoralen Komponente der Endoprothese vorgesehen ist. Auch für die Herstellung der Implantationshilfen werden die bildmäßigen Vorlagen der Schablone durch das "Rapid-Prototyping" zur Bereitstellung entsprechender STL-Modelle umgesetzt, welche bspw. aus Epoxydharz hergestellt werden und dann noch die Führungsschlitze entsprechend der Vorgaben durch die übernommenen markierten Schnittflächen erhalten.

Für die Durchführung der Kniegelenk-Operation kann somit dem behandelnden Arzt ein an dem geschädigten Kniegelenk abgenommenes Operationsset zur Verfügung stehen, bestehend aus femoralen und tibialen Komponenten einer Endoprothese und femoralen und tibialen Komponenten einer zugehörigen Implantationshilfe, hergestellt nach dem vorbeschriebenen erfindungsgemäßen Verfahren. Nach der Öffnung des Kniegelenks werden zuerst an der Tibia und dann anschließend am Femur die geschädigten Gelenkteile abgetrennt bzw. absegmentiert, wobei dafür die Implantationshilfen zur Führung eines oszillierenden Sägeblattes benutzt werden. Es werden daher an den Gelenkknochen Resektionsflächen erhalten, die passgenau übereinstimmen mit den femoralen und tibialen Komponenten der Endoprothese, welche dann unter Ausnutzung der Verankerungshilfen an den Resektionsflächen zementfrei verankert werden können. Bei einem Fehlen von besonderen Verankerungshilfen können die Komponenten der Endoprothese auch direkt mit den Resektionsflächen verklebt werden oder es kann alternativ auch ein sog. Zementier-Verfahren zur Anwendung kommen, um vor einem anschließendem Verschließen bzw. Zunähen des Kniegelenks einen physiologisch einwandfrei festen Sitz für die Komponenten der Endoprothese zu erhalten.

Die Herstellung der Endoprothese kann auch die Bereitstellung einer Komponente beinhalten, die für die Patella des geschädigten Kniegelenks zu verwenden ist. Das Verfahren ist daneben auch anwendbar für die Durchführung von Operationen an anderen Gelenken, so bspw. am Sprunggelenk oder auch an Finger- und Zehengelenken, und es kann auch bei allen Rekonstruktionen von knöchernen und knorpeligen Geweben sowie auch von Weichteilgeweben angewandt werden.

## Patentansprüche

1. Verfahren zur Herstellung einer Endoprothese als Gelenkersatz bei Kniegelenken bei der Durchführung von Kniegelenk-Operationen, bestehend aus den Stufen:
a) Anfertigung eines tomographischen Bildes von dem geschädigten Kniegelenk;
b) virtuelle Korrektur des tomographischen Bildes für eine Annäherung der Konturen wenigstens der femoralen und tibialen Gelenkteile des geschädigten Kniegelenks an deren Konturen bei einem gesunden Kniegelenk;
c) virtuelle Abtrennung der korrigierten femoralen und tibialen Gelenkteile als bildmäßige Vorlagen für die Endoprothese, wobei die Abtrennung an markierten Schnittflächen vorgenommen wird, die bei der späteren Operation für die an den Gelenkknochen vorgenommene Abtrennung der geschädigten Gelenkteile vorgegeben werden;
d) Übernahme der für die virtuelle Abtrennung der korrigierten femoralen und tibialen Gelenkteile markierten Schnittflächen für die Anfertigung eines virtuellen Bildes einer Schablone getrennt für Femur und Tibia des geschädigten Kniegelenks als bildmäßige Vorlage für eine mit dem geschädigten Kniegelenk passgenau übereinstimmende Implantationshilfe, bei welcher die übernommenen Schnittflächen als Führungsschlitze ausgeführt werden, die bei der späteren Operation ein oszillierendes Sägeblatt für die an den Gelenkknochen vorgenommene Abtrennung der geschädigten Gelenkteile führen;
e) Herstellung von dreidimensionalen femoralen und tibialen Komponenten der Endoprothese und von dreidimensionalen femoralen und tibialen Komponenten der zugehörigen Implantationshilfe auf der Basis ihrer bildmäßigen Vorlagen.

2. Verfahren nach Anspruch 1, bei welchem die korrigierten femoralen und tibialen Gelenkteile an den markierten Schnittflächen bildmäßig ergänzt werden mit passgenauen Verankerungshilfen für die späteren dreidimensionalen Komponenten der Endoprothese an den Resektionsflächen der Gelenkknochen.

3. Verfahren nach Anspruch 1 oder 2, bei welchem die tomographischen Bilder mittels der Computer-Tomographie oder der Kernspintomographie angefertigt werden.

4. Verfahren nach einem der Ansprüche 1 bis 3, bei welchem die virtuelle Korrektur des tomographischen Bildes von dem geschädigten Kniegelenk manuell oder mit dem Bild einer Spiegelbildaufnahme eines zu dem geschädigten Kniegelenk gegenseitigen, gesunden Kniegelenks des Patienten vorgenommen wird.

5. Verfahren nach einem der Ansprüche 1 bis 3, bei welchem die virtuelle Korrektur des tomographischen Bildes von dem geschädigten Kniegelenk durch einen Vergleich mit Bildern von gesunden Kniegelenken vorgenommen wird, die mit dem geschädigten Kniegelenk vergleichbare Gelenkflächen wenigstens des Femur und der Tibia aufweisen.

6. Operationsset zur Durchführung von Kniegelenk-Operationen, bestehend aus:
c1) femoralen und tibialen Komponenten einer Endoprothese, die aus tomographisch angefertigtem bildmäßigen Vorlagen von femoralen und tibialen Gelenkteilen erhalten sind, welche an markierten Schnittflächen eines tomographisch angefertigten und korrigierten Bildes von einem geschädigten Kniegelenk virtuell abgetrennt wurden, und
d1) femoralen und tibialen Komponenten einer dreidimensionalen Implantationshilfe, die aus den bildmäßigen Vorlagen einer an den markierten Schnittflächen des korrigierten Bildes von dem geschädigten Kniegelenk ausgerichteten Schablone getrennt für Femur und Tibia erhalten sind, wobei die markierte Schnittfläche bei den beiden femoralen und tibialen Komponenten der Implantationshilfe als Führungsschlitze für ein oszillierendes Sägeblatt ausgebildet sind.

7. Operationsset nach Anspruch 6, bei welchem die femoralen und tibialen Komponenten der Endoprothese mit zapfenförmigen Vorsprüngen für eine Einpassung in Bohrkanäle in den Resektionsflächen der Gelenkknochen an dem geschädigten Kniegelenk versehen sind.

8. Operationsset nach Anspruch 6 oder 7, bei welchem die tibiale Komponente der Endoprothese aus einem Kunststoffteil und einem Metallteil besteht, wobei das Kunststoffteil eine mittige Anordnung zwischen dem Metallteil der tibialen Komponente und der ebenfalls als ein Metallteil ausgeführten femoralen Komponente der Endoprothese aufweist.

## Claims

1. Method of producing an endoprosthesis as articular substitute for knee joints in the realisation of surgery on the knee joint, comprising the following steps:
(a) production of a tomographic image of the damaged knee joint;
(b) virtual correction of the tomographic image for approximating the contours of at least the femoral and tibial articular parts of the damaged knee joint to their contours in an unaffected knee joint;
(c) virtual separation of the corrected articular femoral and tibial parts as image models for the endoprosthesis, with the separation being carried out at marked cut surfaces that are specified for the detachment of damaged joint parts on the articular bones by the time of the subsequent surgical operation;
(d) transfer of the cut surfaces marked for the virtual detachment of the corrected femoral and tibial joint parts for producing a virtual image of a model, separately for the femur and the tibia of the damaged knee joint as image model of an implantation-assisting material congruent with precise fit with the damaged knee joint, wherein the transferred cut surfaces are realised in the form of guiding slots that guide an oscillating saw blade in the subsequent surgical operation for the detachment carried out on the articular bones of the damaged joint parts;
(e) production of the femoral and tibial components of the endoprosthesis and of three-dimensional femoral and tibial components of the corresponding implantation-assisting material on the basis of their image model.

2. Method according to Claim 1, wherein the corrected femoral and tibial articular parts are completed in terms of image at the marked cut surfaces by precisely fitting anchorage-assisting means for the subsequent three-dimensional components of the endoprosthesis at the resection surfaces on the articular bones.

3. Method according to Claim 1 or 2, wherein the tomographic images are produces by computer tomography or nuclear magnetic resonance tomography.

4. Method according to any of the Claims 1 to 3, wherein the virtual correction of the tomographic image of the damaged knee joint is carried out by hand or by means of the image of a mirror-image picture of an unaffected knee joint of the patient, that is opposite to the damaged knee joint.

5. Method according to any of the Claims 1 to 3, wherein the virtual correction of the tomographic image of the damaged knee joint is carried out by a comparison against the images of unaffected knee joints, which present articular surfaces at least of the femur and the tibia, which are comparable to the damaged knee joint.

6. Surgical operation set for carrying out surgery on knee joints, comprising:
(c1) femoral and tibial components of an endoprosthesis, which are derived from image models created by tomography of articular femoral and tibial parts that have been virtually detached at marked cut surfaces of an image of a damaged knee joint, which was created by tomography and corrected, and
(d1) femoral and tibial components of a three-dimensional implantation-assisting material, which are derived from image models of a model oriented towards the cut surfaces of the corrected image of the damaged knee joint, separately for the femur and the tibia, with the cut surfaces on the two femoral and tibial components of the implantation-assisting material being realised in the form of guiding slots for guiding an oscillating saw blade.

7. Surgical operation set according to Claim 6, wherein the femoral and tibial components of the endoprosthesis are provided with spigot-shaped projections for fitting into bores drilled into the resection surfaces of the articular bones on the damaged knee joint.

8. Surgical operation set according to Claim 6 or 7, wherein the tibial component of the endoprosthesis is composed of a synthetic part and a metal part, with the synthetic part presenting a central arrangement between the metal part of the tibial component and the femoral part of the endoprosthesis, which is equally configured in the form of a metal part.

## Revendications

1. Procédé à créer une endoprothèse en tant que substitut articulaire pour des articulations du genou dans la réalisation des opérations chirurgicales sur l'articulation de genou, comprenant les étapes suivantes :
(a) production d'une image tomographique de l'articulation du genou lésée;
(b) correction virtuelle de l'image tomographique pour une approximation des contours d'au moins des parties articulaires fémorales et tibiales de l'articulation du genou lésée à leurs contours dans une articulation du genou saine;
(c) séparation virtuelle des parties articulaires fémorales et tibiales corrigées en tant que modèles en image pour l'endoprothèse, à la séparation se faisant aux aires de coupe marquées, qui sont spécifiées pour l'ablation des parties de l'articulation lésées aux os articulaires au cours de l'opération chirurgicale ultérieure;
(d) reprise pour la séparation virtuelle des aires de coupe marquées pour l'ablation virtuelle des parties articulaires corrigées pour la production d'une image virtuelle d'un modèle, séparée pour le fémur et le tibia de l'articulation du genou lésée en tant que modèle en image d'une aide d'implantation en congruence sur mesure avec l'articulation du genou lésée, dans lequel les aires de coupe reprises sont réalisées sous forme de fentes de guidage, qui guident une lame de scie oscillante dans l'opération chirurgicale ultérieure pour l'ablation faite aux os articulaires des parties articulaires lésées;
(e) production des composants fémorales et tibiales de l'endoprothèse et des composants fémorales et tibiales tridimensionnelles de l'aide d'implantation correspondante sur la base de leurs modèles en image.

2. Procédé selon la revendication 1, dans lequel les parties articulaires fémorales et tibiales corrigées sont complémentées en image, aux aires de coupe marquées, moyennant des aides d'ancrage sur mesure pour les composants tridimensionnels ultérieurs de l'endoprothèse aux aires de résection des os articulaires.

3. Procédé selon la revendication 1 ou 2, dans lequel les images tomographiques sont créées moyennant la scanographie ou la tomographie à résonance magnétique nucléaire.

4. Procédé selon une quelconque des revendications 1 à 3, dans lequel la correction virtuelle de l'image tomographique de l'articulation du genou lésée se fait à la main ou au moyen de l'image d'une vue prise en image spéculaire d'une articulation du genou saine du patient, opposée à l'articulation du genou lésée.

5. Procédé selon une quelconque des revendications 1 à 3, dans lequel la correction virtuelle de l'image tomographique de l'articulation du genou lésée se fait par une comparaison avec des images des articulations du genou saines, qui présentent des aires articulaires au moins du fémur et du tibia, qui sont comparables à l'articulation du genou lésée.

6. Jeu de l'opération chirurgicale à réaliser des opérations chirurgicales aux articulations du genou, comprenant :
(c1) des composants fémorales et tibiales d'une endoprothèse, qui sont dérivés des modèles en image, produits par une méthode tomographique, des parties articulaires fémorales et tibiales, qui étaient séparés de façon virtuelle aux aires de coupe marquées d'une image d'une articulation du genou lésée, qui était produite par une méthode tomographique et corrigée, et
(d1) des composants fémorales et tibiales d'une aide d'implantation tridimensionnelle, qui sont dérivés des modèles en image d'un modèle orienté sur les aires de coupe de l'image corrigée de l'articulation du genou lésée, séparément pour le fémur et le tibia, aux aires de coupe sur les deux composants fémorales et tibiales de l'aide d'implantation étant réalisées sous forme de fentes de guidage pour une lame de scie oscillante.

7. Jeu de l'opération chirurgicale selon la revendication 6, dans lequel les composants fémorales et tibiales de l'endoprothèse sont pourvus des parties en saillie en forme de tenons pour l'ajustement dans des passages alésés dans les aires de résection des os articulaires à l'articulation du genou lésée.

8. Jeu de l'opération chirurgicale selon la revendication 6 ou 7, dans lequel le composant tibial de l'endoprothèse est constitué par une partie en résine synthétique et une partie métallique, à la partie en résine synthétique présentant un arrangement central entre la partie métallique du composant tibial et le composant fémoral de l'endoprothèse, qui est également configuré sous forme d'une partie métallique.
